Europäisches Patentamt

European Patent Office

Office européen des brevets

(11) Veröffentlichungsnummer : **0 427 062 B1**

# EUROPÄISCHE PATENTSCHRIFT

(45) Veröffentlichungstag der Patentschrift :
**16.02.94 Patentblatt 94/07**

(51) Int. Cl.$^5$ : **C07C 67/40,** C07C 69/06

(21) Anmeldenummer : **90120549.2**

(22) Anmeldetag : **26.10.90**

(54) **Verfahren zur Herstellung von Methylformiat.**

Die Akte enthält technische Angaben, die nach dem Eingang der Anmeldung eingereicht wurden und die nicht in dieser Patentschrift enthalten sind.

(30) Priorität : **06.11.89 DE 3936854**

(43) Veröffentlichungstag der Anmeldung :
**15.05.91 Patentblatt 91/20**

(45) Bekanntmachung des Hinweises auf die Patenterteilung :
**16.02.94 Patentblatt 94/07**

(84) Benannte Vertragsstaaten :
**BE DE ES FR GB IT NL SE**

(56) Entgegenhaltungen :
**DE-A- 2 753 634**
**US-A- 1 975 853**
**CHEMICAL ABSTRACTS, vol. 89, no. 17, 23. Oktober 1978, Columbus, Ohio, US; abstract no. 146444G, H. YOKOYAMA ET. AL.: 'Methyl Formate' Seite 567 ;Spalte 2 ;**

(73) Patentinhaber : **BASF Aktiengesellschaft**
**Carl-Bosch-Strasse 38**
**D-67063 Ludwigshafen (DE)**

(72) Erfinder : **Joerg, Klaus, Dr.**
**Albert-Einstein-Allee 35**
**W-6703 Limburgerhof (DE)**
Erfinder : **Mueller, Franz-Josef, Dr.**
**Müller-Thurgau-Weg 1**
**W-6706 Wachenheim (DE)**
Erfinder : **Irgang, Matthias, Dr.**
**Andreas-Hofer-Weg 41**
**W-6900 Heidelberg (DE)**
Erfinder : **Marosi, Laszlo, Dr.**
**Leuschnerstrasse 32**
**W-6700 Ludwigshafen (DE)**
Erfinder : **Borchert, Gerhard**
**Londoner Ring 9**
**W-6700 Ludwigshafen (DE)**

**Beschreibung**

Die vorliegende Erfindung betrifft ein Verfahren zur Herstellung von Methylformiat durch die dehydrierende Umsetzung von Methanol an kupferhaltigen Katalysatoren bei erhöhter Temperatur und in der Gasphase.

Methylformiat wird zum größten Teil durch die Umsetzung von Methanol mit Kohlenmonoxid in Gegenwart von Alkalimetallalkoholaten hergestellt. Für die wirtschaftliche Durchführbarkeit dieses Verfahrens ist es jedoch wichtig, Anlagen zur Erzeugung und Lieferung ausreichender Mengen an Kohlenmonoxid am Standort der Methylformiatproduktion oder in dessen Nähe zu haben. Dies ist aber insbesondere bei kleineren Chemieproduktionsstandorten oftmals nicht der Fall, weshalb für diese Standorte Alternativsynthesen für die Herstellung von Methylformiat, welche ohne Kohlenmonoxid auskommen - beispielsweise die dehydrierende Umsetzung von Methanol zu Methylformiat - wirtschaftlich interessant sind.

Die dehydrierende Umsetzung von Methanol zu Methylformiat ist seit längerer Zeit bekannt, beispielsweise aus US-A 1 400 195, US-A 1 975 853, JP-A 68716/1978 und US-A 2 160 064. Dabei werden Katalysatoren aus metallischem Kupfer, zum Teil in Form von Trägerkatalysatoren eingesetzt. Die Wirtschaftlichkeit dieser Verfahren war jedoch unbefriedigend, weswegen sich die Verfahren dieser Patentschriften nicht durchsetzen konnten.

In den vergangenen Jahren wurde versucht, dieses Verfahren durch die Entwicklung neuer Katalysatoren wirtschaftlicher zu gestalten. So werden in DE-A 27 16 842 Katalysatoren zur Dehydrierung von Methanol zu Methylformiat beschrieben, welche neben Kupfer wenigstens ein Element aus einer der Gruppen IIIA, einschließlich der Seltenerdmetalle, der Gruppe IVA sowie der Gruppe der Aktiniden, enthalten. Der Gehalt an den relativ seltenen Elementen der Gruppe IIIA und der Seltenerdmetalle macht diese Katalysatoren unwirtschaftlich teuer und der Gebrauch von Aktiniden als Katalysatorbestandteil ist im industriellen Maßstab allein schon aus Gründen des Strahlenschutzes indiskutabel.

In DE-A 27 53 634 ist die dehydrierende Umsetzung von Methanol zu Methylformiat mit Kupferkatalysatoren publiziert, welche zusätzlich Zirkonium, Zink und wahlweise Aluminium enthalten. Nach den Angaben dieser Schrift sollen Kupfer/Zink/Zirkonium-Katalysatoren und Kupfer/Zink/Zirkonium/Aluminium-Katalysatoren, in denen diese Elemente zueinander in Atomverhältnissen von 1/0,3/0,3 bzw. 1/0,3/0,3/0,1 vorliegen, eine Selektivität bei der Herstellung von Methylformiat von 83,6 bzw. 84,6 % bei Methylformiatausbeuten von 48,9 bzw. 62,0 % und bei einer Temperatur von 280°C erlauben. Bei der Nacharbeitung dieser Versuche konnten mit diesen Katalysatoren bei der Herstellung von Methylformiat aus Methanol in den besten Fällen allerdings nur Selektivitäten von 2,2 bzw. 43,5 % bei Ausbeuten von 1,5 bzw. 28,5 % erzielt werden. Das Leistungsvermögen dieser Katalysatoren ist somit für eine wirtschaftliche Ausführung dieses Verfahrens zur Herstellung von Methylformiat nicht ausreichend.

Es bestand somit die Aufgabe, ein Verfahren zur Verfügung zu stellen, das die Herstellung von Methylformiat durch die dehydrierende Umsetzung von Methanol auf wirtschaftliche Weise ermöglicht. Zu diesem Zweck sollten Katalysatoren gefunden werden und zum Einsatz kommen, welche auf einfache Weise aus leicht und kostengünstig verfügbaren Ausgangsmaterialien erhältlich sind.

Dementsprechend wurde ein Verfahren zur Herstellung von Methylformiat durch Umsetzung von Methanol an kupferhaltigen Katalysatoren bei erhöhter Temperatur und in der Gasphase gefunden, das dadurch gekennzeichnet ist, daß man einen Katalysator verwendet, der neben 30 bis 80 Gew.-% Kupfer, berechnet als CuO, 20 bis 70 Gew.-% Magnesiumsilikate, berechnet als MgO plus $SiO_2$, enthält, in denen das Molverhältnis $MgO/SiO_2$ 0,3 bis 1,5 beträgt und der zusätzlich mit den Metallen Zink, Chrom und/oder Barium in Mengen von jeweils bis zu 1,5 Gew.-%, berechnet als ZnO, $Cr_2O_3$ und BaO, dotiert sein kann.

Die erfindungsgemäß verwendeten Katalysatoren enthalten als Hauptbestandteile Kupfer und Magnesiumsilikate und können weiterhin noch mit geringen Mengen an Zink, Chrom und/oder Barium dotiert sein. Die im folgenden genannten Mengenangaben beziehen sich jeweils, falls nichts anderes angemerkt ist, auf den fertigen, glühverlustfreien Katalysator vor dessen Reduktion mit Wasserstoff.

Die erfindungsgemäß verwendeten Katalysatoren haben im allgemeinen Kupfergehalte von 30 bis 80, vorzugsweise 40 bis 80 und insbesondere 60 bis 80 Gew.-%, berechnet als CuO. Die Magnesiumsilikate liegen in diesen Katalysatoren in Mengen von 20 bis 70, bevorzugt von 20 bis 60 und insbesondere von 20 bis 40 Gew.-% vor und zwar berechnet als MgO plus $SiO_2$. Die Katalysatoren werden so hergestellt, daß das Molverhältnis $MgO/SiO_2$ im Katalysator im allgemeinen 0,3 bis 1,5, vorteilhaft 0,5 bis 1,2 und bevorzugt 0,6 bis 0,9 beträgt. Des weiteren können die erfindungsgemäß verwendeten Katalysatoren mit jeweils bis zu 1,5, vorteilhaft 0,3 bis 1,3 und insbesondere mit 0,6 bis 1,1 Gew.-% der Metalle Zink, Chrom und/oder Barium, berechnet als ZnO, $Cr_2O_3$ und BaO dotiert sein.

Somit können im erfindungsgemäßen Verfahren Katalysatoren zur Anwendung gelangen, welche nur Kupfer und Magnesiumsilikate enthalten, es können vorteilhaft aber auch Katalysatoren eingesetzt werden, die mit einem oder mehreren, vorzugsweise mit allen der genannten übrigen Metalle innerhalb der genannten

2

Grenzen dotiert sind.

Die Katalysatoren werden im allgemeinen hergestellt, in dem man Magnesiumsilikat durch Zugabe einer Magnesiumsalzlösung, vorzugsweise einer Magnesiumnitratlösung zu einer zweckmäßigerweise stark alkalischen Alkalimetallsilikatlösung, vorzugsweise einer Wasserglaslösung, ausfällt, wobei die Mengenverhältnisse der umzusetzenden Salzlösungen vorteilhafterweise so gewählt werden, daß der sich bildende Niederschlag Magnesium und Silikat, berechnet als MgO und SiO$_2$, im gewünschten Molverhältnis enthält. Die bei der Umsetzung der Magnesiumsalzlösung mit der Wasserglaslösung erhaltene Fällung ist röntgenamorph.

Zu der erhaltenen Magnesiumsilikatsuspension werden dann, zweckmäßigerweise unter Rühren, das Kupfer und gewünschtenfalls die Komponenten Zink, Chrom und/oder Barium in Form ihrer Salzlösungen, vorzugsweise in Form ihrer Nitratlösungen, zugegeben. Das Fällungsmittel kann vor oder nach der Zugabe dieser Metallsalzlösung zu dieser Mischung dosiert werden, vorteilhaft wird es aber gleichzeitig mit den Metallnitratlösungen in die Fällungsapparatur eingeleitet. Als Fällungsmittel werden im allgemeinen wäßrige Lösungen der Hydroxide, Carbonate oder Hydrogencarbonate der Alkalimetalle verwendet. Besonders bevorzugt ist der Gebrauch von Natrium- und Kaliumcarbonatlösungen als Fällungsmittel. Die Fällung kann bei Raumtemperatur als auch in der Wärme oder Siedehitze vorgenommen werden. Vorteilhaft wird bei 30 bis 70°C gefällt.

Man kann bei der Herstellung der erfindungsgemäß verwendeten Katalysatoren auch so vorgehen, daß man das Magnesiumsilikat und die übrigen Katalysatorkomponenten separat ausfällt und die erhaltene Niederschläge vor oder nach der Trocknung vermischt, bevorzugt ist jedoch die oben beschriebene Vorgehensweise bei der die genannten Katalysatorkomponenten auf das vorgefällte Magnesiumsilikat aufgefällt werden.

Bei der Herstellung zinkhaltiger Katalysatoren unter Verwendung von Alkalimetallhydroxidlösungen als Fällungsmittel ist selbstverständlich darauf zu achten, daß das ausgefällte Zinkhydroxid nicht infolge der Zugabe zu großer Mengen an Alkalimetallhydroxid wieder als Zinkat in Lösung geht. Bei der Herstellung chromhaltiger Katalysatoren wird vorzugsweise von wasserlöslichen Chrom(III)salzen ausgegangen.

Der auf diese Weise erhaltene, im allgemeinen chemisch uneinheitliche Niederschlag der Katalysatorkomponenten besteht, je nach angewandtem Fällungsmittel, aus einem Gemisch der schwerlöslichen Hydroxide, Carbonate, Oxidhydrate und basischen Salze der gefällten Metallkomponente mit dem bereits vorher gefällten Magnesiumsilikatgemisch.

Die gefällten Katalysatorkomponenten können auf übliche Weise, beispielsweise durch Filtration oder Zentrifugation von der flüssigen Phase abgetrennt, gewaschen und getrocknet werden. Die Trocknungstemperatur beträgt im allgemeinen 80 bis 200, vorzugsweise 100 bis 160, insbesondere 110 bis 130°C. Nach der Trocknung wird der Katalysator noch kalziniert und zwar im allgemeinen bei Temperaturen von 200 bis 500°C. Zur Herstellung von Katalysatorformkörpern wird die kalzinierte Katalysatorrohmasse zweckmäßigerweise mit Formhilfsmitteln wie Graphit oder Stearinsäure feinvermengt und anschließend zu den gewünschten Formkörpern verpreßt.

Vor ihrem Einsatz zur dehydrierenden Umsetzung von Methanol zu Methylformiat werden die Katalysatoren zweckmäßigerweise im Wasserstoffstrom bei erhöhter Temperatur reduziert. Der Wasserstoff wird bevorzugt als Gasgemisch aus Wasserstoff und Inertgasen, wie Stickstoff oder Argon, angewandt. Die Reduktion des Katalysators wird im allgemeinen bei Temperaturen von 150 bis 250°C ausgeführt. Bei der Reduktion werden die durch Wasserstoff reduzierbaren Metallkomponenten, soweit sie mit dem Reduktionsmittel durch direkten Kontakt oder durch Diffusion in Berührung kommen, zu den Metallen reduziert. Die erfindungsgemäß verwendbaren Katalysatoren können als Pulver oder als Formkörper zur Umsetzung eingesetzt werden.

Zur Herstellung von Methylformiat nach dem erfindungsgemäßen Verfahren wird Methanol gasförmig bei Temperaturen von im allgemeinen 150 bis 300, vorzugsweise 200 bis 290 und insbesondere bei 240 bis 280°C und bei Drucken von im allgemeinen 0,1 bis 10 bar, vorteilhaft bei Atmosphärendruck über einen der erfindungsgemäß verwendbaren Katalysatoren geleitet und dabei dehydrierend zu Methylformiat umgesetzt.

Die Umsetzung erfolgt im allgemeinen kontinuierlich in Rohrreaktoren oder Wirbelbettreaktoren. Die Katalysatorschüttung wird unter den oben angegebenen Reaktionsbedingungen mit gasförmigem Methanol mit einer Raumgeschwindigkeit von im allgemeinen 100 bis 10000 h$^{-1}$, vorteilhaft von 3500 bis 7000 h$^{-1}$ belastet. Gewünschtenfalls kann der gasförmige Methanolstrom noch durch Inertgase wie Argon oder vorzugsweise Stickstoff verdünnt sein.

Dazu wird das Methanol im allgemeinen in einem dem Reaktor vorgeschalteten Verdampfer, vorteilhaft einer Kolonne, verdampft und auf die Reaktionstemperatur gebracht. Die Katalysatorschüttung kann ebenfalls durch Beheizung von außen auf die Reaktionstemperatur vorgewärmt und während der Umsetzung auf der gewünschten Reaktionstemperatur gehalten werden.

Die Aufarbeitung des den Reaktor verlassenden Reaktionsgemisches, das außer aus Methylformiat, im wesentlichen noch aus nicht umgesetztem Methanol, Wasserstoff und gegebenenfalls Inertgasen besteht, kann nach herkömmlichen Methoden erfolgen. So werden die flüssigen Bestandteile des Reaktionsgemisches - Methylformiat und Methanol - von den gasförmigen Bestandteilen - Inertgase, Wasserstoff, gasförmige Nebenprodukte wie Kohlenmonoxid - zweckmäßiger durch Destillation unter erhöhtem Druck in Gas-Flüssig-Abscheidern abgetrennt. Nach dieser Trennung des Reaktionsaustrags in seine flüssigen und gasförmigen Bestandteile wird das gebildete Methylformiat vom nicht umgesetzten Methanol destillativ abgetrennt und isoliert. Das dabei zurückgewonnene Methanol kann je nach Wunsch in die Umsetzung zurückgeführt oder anderweitigen Verwendungszwecken zugeführt werden.

Nach dem erfindungsgemäßen Verfahren kann Methylformiat durch dehydrierende Umsetzung von Methanol auf wirtschaftliche Weise hergestellt werden.

Methylformiat dient im wesentlichen zur Herstellung von Ameisensäure und den verschiedenen Formamiden.

Beispiele

Katalysatorherstellung

Katalysator 1:

Eine stark alkalische Natriumwasserglas-Lösung und eine Magnesiumnitrat-Lösung wurden bei 35°C unter Rühren vermischt, wobei ein Magnesiumsilikatniederschlag mit einem Molverhältnis $MgO/SiO_2$ von 0,7 entstand. Zu der erhaltenen Magnesiumsilikat-Suspension wurde unter Rühren und bei 55°C eine Mischlösung aus Kupfer(II)nitrat, Zinknitrat, Chrom(II)nitrat und Bariumnitrat zugepumpt. Gleichzeitig mit dieser Lösung wurde Natriumcarbonatlösung in die Suspension eingeleitet. Der erhaltene Niederschlag wurde abfiltriert, gewaschen und bei 120°C getrocknet. Bei 250°C wurde er anschließend so lange kalziniert bis der Glühverlust ca. 15 Gew.-% betrug. Nach Zumischung von 3 Gew.-% Graphit als Formhilfsmittel wurde die erhaltene Masse zu Tabletten gepreßt.

Der auf diese Weise hergestellte Katalysator enthielt 77,4 Gew.-% Kupfer, berechnet als CuO, 0,3 Gew.-% Chrom, berechnet als $Cr_2O_3$, 0,3 Gew.-% Zink, berechnet als ZnO, 0,4 Gew.-% Barium, berechnet als BaO, 7,0 Gew.-% Magnesium, berechnet als MgO und 14,1 Gew.-% Silikat, berechnet als $SiO_2$. Das Schüttgewicht des Katalysators betrug 820 g/l, die Porosität, gemessen als Wasseraufnahme, 0,39 ml/g und die Stirndruckfestigkeit 4200 $N/cm^2$.

Katalysator 2:

Analog dem Verfahren zur Herstellung von Katalysator 1 wurde ein Katalysator hergestellt, der nach der Kalzinierung und vor seiner Reduktion, die folgende chemische Zusammensetzung hatte:
44,8 Gew.-% Kupfer, berechnet als CuO,
0,7 Gew.-% Chrom, berechnet als $Cr_2O_3$,
0,5 Gew.-% Zink, berechnet als ZnO,
1,2 Gew.-% Barium, berechnet als BaO,
17,7 Gew.-% Magnesium, berechnet als MgO,
35,0 Gew.-% Silikat, berechnet als $SiO_2$.

Vergleichskatalysator A

Der Vergleichskatalysator A wurde gemäß Beispiel 1 von DE-A 27 16 842 durch Verkneten von Kupfercarbonat und Zirkoniumcarbonat mit Wasser, Trocknung, Temperung und Formung mit Hilfe von 3 Gew.-% Graphit als Formhilfsmittel, erhalten. Der Katalysator enthielt 87 Gew.-% Kupfer, berechnet als CuO und 13 Gew.-% Zirkonium, berechnet als $ZrO_2$. Sein Schüttgewicht betrug 2110 g/l, seine Wasseraufnahme 0,14 ml/g und seine Stirndruckfestigkeit 1610 $N/cm^2$.

Vergleichskatalysator B

Vergleichskatalysator B wurde gemäß Beispiel 1 von DE-A 27 53 634 durch Fällung einer Zinknitrat-/Zirkoniumacetat-Lösung mit Natriumcarbonatlösung, Filtration, Auswaschung und Vermengung des erhaltenen Niederschlages mit Kupfercarbonat, Trocknung, Temperung und Tablettierung erhalten. Der Katalysator enthielt 56,5 Gew.-% Kupfer, berechnet als CuO, 17,3 Gew.-% Zink, berechnet als ZnO und 26,2 Gew.-% Zirkonium, berechnet als $ZrO_2$. Sein Schüttgewicht betrug 1461 g/l, die Wasseraufnahme 0,23 ml/g und seine Stirndruckfestigkeit 3000 $N/cm^2$.

4

Vergleichskatalysator C

Vergleichskatalysator C wurde gemäß Beispiel 2 von DE-A 27 53 634 durch Fällung einer Kupfernitrat-/Zinknitrat-Lösung mit Natriumcarbonatlösung, Filtration, Auswaschung und Vermischung des erhaltenen Niederschlags mit Aluminiumhydroxid und Zirkoniumcarbonat, Trocknung, Temperung und Tablettierung erhalten. Der fertige Katalysator enthielt 50,6 Gew.-% Kupfer, berechnte als CuO, 21,9 Gew.-% Zink, berechnet als ZnO, 3,4 Gew.-% Aluminium, berechnet als $Al_2O_3$ und 23 Gew.-% Zirkonium, berechnet als $ZrO_2$. Sein Schüttgewicht betrug 1810 g/l, die Wasseraufnahme 0,15 ml/g und seine Stirndruckfestigkeit 4028 $N/cm^2$.

Herstellung von Methylformiat

Die oben angeführten Katalysatoren wurden zu Splitt einer Korngröße von 1,5 bis 1,8 mm zerkleinert. Jeweils 30 ml dieser Katalysatoren wurden in einen Quarzglasreaktor mit einem Innendurchmesser von 20 mm eingefüllt und mit einem Wasserstoff-/Stickstoff-Gasgemisch ($H_2/N_2$: 2/98 Vol.-%) bei 200°C 16 h lang reduziert.

Über die reduzierten Katalysatoren wurde bei Atmosphärendruck bei den in den Tabellen angegebenen Temperaturen (T) und mit den angegebenen Raumgeschwindigkeiten (RG) Methanoldampf geleitet.

Die Zusammensetzung des Reaktionsaustrages wurde gaschromatographisch ermittelt und aus den Ergebnissen dieser Analyse der Methanolumsatz (U) sowie die Selektivität (S) für Methylformiat errechnet. Die Versuchsergebnisse sind in den Tabellen 1 bis 3 aufgelistet.

Tabelle 1: Ergebnisse der Methylformiatherstellung mit Katalysator 1

| Temperatur °C | RG $h^{-1}$ | U(MeOH) % | S(Mefo) % |
|---|---|---|---|
| 180 | 5 800 | 6.8 | 98.0 |
| 200 | 6 062 | 14.1 | 83.3 |
| 220 | 6 319 | 17.8 | 99.0 |
| 240 | 6 575 | 26.3 | 94.2 |
| 260 | 6 831 | 36.2 | 87.4 |
| 260 | 5 960 | 42.7 | 75.6 |

MeOH: Methanol
Mefo: Methylformiat

Tabelle 2: Ergebnisse der Methylformiatherstellung mit Katalysator 2

| Temperatur °C | RG h-1 | U(MeOH) % | S(Mefo) % |
|---|---|---|---|
| 160 | 3 600 | 20.8 | 75.3 |
| 180 | 3 871 | 29.9 | 71.1 |
| 200 | 4 042 | 36.1 | 55.0 |
| 220 | 4 213 | 41.0 | 48.1 |
| 180 | 5 807 | 27.0 | 92.3 |
| 200 | 6 068 | 35.9 | 66.1 |

Tabelle 3: Ergebnisse der Methylformiatherstellung mit den
Vergleichskatalysatoren A, B und C

| Vergleichs-katalysator | Temperatur °C | RG h-1 | U(MeOH) % | S(Mefo) % |
|---|---|---|---|---|
| A | 180 | 2 700 | 3 | 55 |
| A | 200 | 2 830 | 2.4 | 87.2 |
| A | 220 | 2 940 | 1.6 | 76.0 |
| A | 240 | 3 040 | 4 | 12.9 |
| A | 260 | 3 190 | 4.1 | 38.1 |
| A | 280 | 3 310 | 8 | 60 |
| A | 300 | 3 410 | 14.1 | 69.3 |
| A | 310 | 3 460 | 19.1 | 72.7 |
| B | 180 | 3 900 | 1.6 | 81.4 |
| B | 200 | 4 150 | 3.1 | 82.7 |
| B | 240 | 4 430 | 6.1 | 90.1 |
| B | 260 | 4 650 | 27.3 | 78.7 |
| B | 280 | 4 820 | 43.3 | 74.7 |
| B | 300 | 5 080 | 62.1 | 31.4 |
| B | 310 | 5 210 | 77.0 | 16.5 |
| C | 240 | 6 600 | 16.3 | 76.0 |
| C | 260 | 7 060 | 24.2 | 57.9 |
| C | 280 | 7 270 | 36.9 | 32.9 |
| C | 300 | 7 380 | 49.1 | 15.7 |
| C | 310 | 7 470 | 53.3 | 5.9 |

**Patentansprüche**

1. Verfahren zur Herstellung von Methylformiat durch dehydrierende Umsetzung von Methanol an kupferhaltigen Katalysatoren bei erhöhter Temperatur und in der Gasphase, dadurch gekennzeichnet, daß man einen Katalysator verwendet, der neben 30 bis 80 Gew.-% Kupfer, berechnet als CuO, 20 bis 70 Gew.-% Magnesiumsilikate, berechnet als MgO plus $SiO_2$, enthält, in denen das Molverhältnis $MgO/SiO_2$ 0,3 bis 1,5 beträgt und der zusätzlich mit den Metallen Zink, Chrom und/oder Barium in Mengen von jeweils bis zu 1,5 Gew.-%, berechnet als ZnO, $Cr_2O_3$ und BaO, dotiert sein kann.

2. Verfahren nach Anspruch 1, dadurch gekennzeichnet, daß das Methanol bei einer Temperatur von 150 bis 300°C und bei einem Druck von 0,1 bis 10 bar über den Katalysator geleitet wird.

**Claims**

1. A process for the preparation of methyl formate by dehydrogenating methanol over a copper-containing catalyst at elevated temperatures and in the gas phase, wherein the catalyst used contains from 30 to 80% by weight of copper, calculated as CuO, as well as from 20 to 70% by weight of a magnesium silicate, calculated as MgO plus $SiO_2$, which has a molar ratio of $MgO/SiO_2$ of from 0.3 to 1.5, and may additionally be doped with the metals zinc, chromium and/or barium, each in an amount of not more than 1.5% by weight, calculated as ZnO, $CrO_2O_3$ and BaO.

2. A process as claimed in claim 1, wherein the methanol is passed over the catalyst at from 150 to 300°C and at from 0.1 to 10 bar.

## Revendications

1. Procédé de préparation de formiate de méthyle par réaction déshydrogénante du méthanol sur des catalyseurs contenant du cuivre à température élevée et en phase gazeuse, caractérisé en ce qu'on utilise un catalyseur qui contient, outre 30 à 80% en poids de cuivre, exprimé en CuO, 20 à 70% en poids de silicate de magnésium exprimé en MgO plus $SiO_2$, dans lequel le rapport molaire $MgO/SiO_2$ est compris entre 0,3 et 1,5 et qui peut en outre être dopé avec les métaux zinc, chrome et/ou baryum en quantités à chaque fois allant jusqu'à 1,5% en poids, exprimées en ZnO, $Cr_2O_3$ et BaO.

2. Procédé selon la revendication 1, caractérisé en ce que l'on charge le méthanol sur le catalyseur à une température de 150 à 300°C et sous une pression de 0,1 à 10 bar.